# EUROPEAN PATENT APPLICATION

(11) **EP 1 806 129 A1**
(43) Date of publication of application: **11.07.2007**
(21) Application number: 05787703.7
(22) Date of filing: 30.09.2005
(51) Int. Cl.: A61K 9/14, A61K 9/10, A61K 9/16, A61K 9/20, A61K 9/48, A61K 47/26, A61K 47/40

(54) **COMPOSITION CONTAINING FINE PARTICLES AND PROCESS FOR PRODUCING THE SAME**

(30) Priority: 01.10.2004 JP 2004289680
(71) Applicant: Eisai R&D Management Co., Ltd., Tokyo 112-8088 (JP)
(72) Inventor: UEKI, Yosuke c/o Kawashima Industrial Complex,, Kakamigahara-shi, Gifu 5016195 (JP)
(74) Representative: Smyth, Gyles Darren
(86) International application number: PCT/JP2005/018099
(87) International publication number: WO 2006/038552

(57) **Abstract**

The present invention provides a composition containing fine particles of a hardly-soluble drug which is stable and is not affected by storage environment conditions, as well as a manufacturing method therefor. The present invention provides a fine particle-containing composition containing fine particles of the hardly-soluble drug, a surfactant and a cyclic oligosaccharide, wherein an average particle size of the fine particles is at least 50 nm but not more than 1000 nm. The present invention also provides a method for manufacturing a fine particle-containing composition, comprising (I) a mixing step in which the hardly-soluble drug, the surfactant and a poor solvent are mixed to obtain a liquid mixture, (II) a pulverization step in which the liquid mixture is pulverized with a wet disperser to obtain a dispersion of fine particles, (III) an addition step in which the cyclic oligosaccharide is added to the dispersion of fine particles, and (IV) a drying step in which the dispersion of fine particles containing the cyclic oligosaccharide is dried.

## Description

### Technical Field

The present invention relates to a composition containing fine particles of a hardly-soluble drug, and to a manufacturing method therefor.

### Background Art

Hardly-soluble drugs are generally difficult to formulate as injections because they dissolve only slightly in water. When mixed in solid preparations, moreover, they do not elute easily from the solid preparations, detracting from bioavailability. Efforts have been made to resolve these problems by making hardly-soluble drugs finer and therefore more easily dispersible in water. For example, injections containing nano-sized anti-cancer drugs and the like are known to exhibit low toxicity as well as high bioavailability.

The most popular method of manufacturing fine particles of a hardly-soluble drug is wet pulverization using water or an organic solvent, and surfactants and other surface modifiers are also used during wet pulverization. This is because surfactants and the like contribute static or steric repulsion and the like to the particle surfaces of the hardly-soluble drug, thereby preventing the particles from aggregating in liquid and making it easier to maintain uniform dispersibility in suspension. Moreover, clouding point adjusters are also used so that the function of the surfactant is not lost when a suspension containing fine particles of the hardly-soluble drug dispersed with a surfactant is treated at high temperatures in an autoclave or the like. For example, a combination of an ionic surfactant with a tonicity agent such as mannitol, dextrose or sodium chloride (see for example US Patent No. 5,298,262, Specifications) and a combination of a nonionic surfactant with a clouding point adjuster such as a glycol, ethanol or hydroxypropyl cyclodextrin (see for example US Patent No. 5,346,702, Specifications) have been disclosed. A stable injection has also been obtained even with high-temperature treatment in an autoclave or the like by using a nonionic surfactant such as pluronic and a polymer substance (see for example Japanese Patent Application Publication 2002-538199) to limit the particle size of the hardly-soluble drug to 150 to 350 nm.

A method has also been disclosed wherein a suspension obtained by the aforementioned method of manufacturing particles of a hardly-soluble drug is further dried by freeze-drying, spray-drying or fluidized bed granulation in order to obtain a composition containing nano-sized particles of a hardly-soluble drug. For example, an injectable freeze-dried preparation having the cryoprotective agent sucrose and the surface stabilizer polyvinylpyrrolidone as essential components has been disclosed which is obtained by a manufacturing method using freeze drying (see for example US Patent No. 5,302,401, Specifications).

However, the desired freeze-dried preparation has not been obtained with a solid composition using a nonionic surfactant or a sugar alcohol such as mannitol. As a manufacturing method using spray-drying, a method has been disclosed wherein a HER2 inhibitor suspension containing hydroxypropyl cellulose and sodium deoxycholate is prepared and then spray-dried (see for example Japanese Patent Application Laid-open No. 2003-26676). As a result, the size of the drug particles in solution and the size of the drug particles after drying remained the same at about 900 nm. As a method using fluidized bed granulation, a method has been disclosed of first preparing a suspension comprising fine particles 500 to 1500 nm in size using a polymer compound, surfactant or sugar as a surface modifier for the drug particles, and then granulating the suspension in a fluidized bed to obtain a solid composition (see for example Japanese Patent Application Laid-open No. 2004-175795).

### Disclosure of Invention

### Problems to be Solved by the Invention

As discussed above, pulverization techniques for hardly-soluble drugs mainly involve applications to injections containing water and other media with the aim of improving the dispersion stability of the fine particles in liquid, while applications to commonly-used solid preparations have not been adequately studied. Although the storage stability of the fine particles in liquid is taken into account in prior art, there is no manufacturing method or composition of fine particles of the hardly-soluble drug that takes into account the stability of the fine particles in a solid composition. Specifically, in conventional methods of manufacturing fine particles of hardly-soluble drugs a surfactant is essential for modifying the surface of the hardly-soluble drug and improving its dispersibility in liquid. A surfactant may not be necessary when the drug is dispersed in a solid preparation, however, and may in fact create problems by promoting crystal growth of the fine particles and fusion between particles.

Moreover, the chemical and physical stability and general quality of pharmaceutical products need to be ensured during the distribution process. This is because chemical and physical changes in a drug or composition during storage not only prevent the product from being used appropriately, but may also adversely impact the safety of the patient. In the case of compositions containing fine particles of a hardly-soluble drug, changes in particle size may detract from the dispersibility of the fine particles in water, adversely affecting bioavailability.

### Means for Solving the Problems

After studying these issues, the inventors discovered that a composition in which crystal growth and re-aggregation of fine particles of a hardly-soluble drug was suppressed and the size of the fine particles maintained during the drying process or other solidification process and even during storage under high-temperature and high-humidity conditions after solidification could be obtained by further adding a cyclic oligosaccharide to a suspension of fine particles (hereinafter referred to as a fine particle dispersion) of a hardly-soluble drug which has been wet pulverized in a solution containing the hardly-soluble drug and a surfactant. They perfected the present invention when they also discovered a method of stabilizing fine particles of the hardly-soluble drug in a fine particle-containing composition by mixing a cyclic oligosaccharide together with a small amount of a poor solvent for the hardly-soluble drug into a fine particle-containing composition obtained by drying a dispersion of fine particles of the hardly-soluble drug which has been wet pulverized in a solution containing the hardly-soluble drug and a surfactant. These inventions provide novel means for suppressing the effects (such as dissolution and fusion) of the surfactant on the fine particles of the hardly-soluble drug in the solid composition because the surfactant is clathrated in the cyclic oligosaccharide.

That is, the present invention provides:
1. a fine particle-containing composition comprising a fine particle of a hardly-soluble drug, a surfactant and a cyclic oligosaccharide, wherein the fine particle has an average particle size in a range of from 50 nm to 1000 nm,
2. the fine particle-containing composition according to item 1, which is manufactured by a manufacturing method comprising the steps of: (I) mixing the hardly-soluble drug, the surfactant and the poor solvent to obtain a liquid mixture; (II) pulverizing the liquid mixture in a wet disperser to obtain a fine particle dispersion; (III) adding the cyclic oligosaccharide to the fine particle dispersion; and (IV) drying the fine particle dispersion containing the cyclic oligosaccharide,
3. the fine particle-containing composition according to item 1, which is manufactured by a manufacturing method comprising the steps of: (I) mixing the hardly-soluble drug, the surfactant, the poor solvent and the cyclic oligosaccharide to obtain a liquid mixture; (II) pulverizing the liquid mixture in a wet disperser to obtain a fine particle dispersion; and (III) drying the fine particle dispersion,
4. the fine particle-containing composition according to item 1, which is manufactured by a manufacturing method comprising the steps of: (I) mixing the hardly-soluble drug, the surfactant and a first poor solvent to obtain a liquid mixture; (II) pulverizing the liquid mixture in a wet disperser to obtain a fine particle dispersion; (III) first drying the fine particle dispersion to obtain a first mixture; (IV) adding a cyclic oligosaccharide and a second poor solvent to the first mixture to obtain a second mixture; and (V) second drying the second mixture,
5. the fine particle-containing composition according to any one of items 2 to 4, wherein the hardly-soluble drug is mixed in the mixing step as a solution of the hardly-soluble drug dissolved in a good solvent,
6. the fine particle-containing composition according to any one of items 2 to 5, wherein the manufacturing method comprises concentrating the fine particle dispersion or the fine particle dispersion containing the cyclic oligosaccharide prior to the drying step or the first drying step,
7. the fine particle-containing composition according to any one of items 2 to 6, wherein the drying step or the first drying step is a drying step employing a spray-drying,
8. the fine particle-containing composition according to any one of items 2 to 7, wherein the wet disperser is a homogenizer,
9. the fine particle-containing composition according to any one of items 1 to 8, wherein the surfactant is clathrated by the cyclic oligosaccharide,
10. the fine particle-containing composition according to any one of items 1 to 9, wherein the cyclic oligosaccharide comprises a cyclodextrin,
11. the fine particle-containing composition according to any one of items 1 to 10, wherein the surfactant comprises a surfactant having a hydrocarbon chain with 4 or more carbon atoms,
12. the fine particle-containing composition according to any one of items 1 to 11, wherein an amount of the hardly-soluble drug is from 0.1 to 40 parts by weight based on 100 parts by weight of the fine particle-containing composition,
13. a solid pharmaceutical composition comprising the fine particle-containing composition according to any one of items 1 to 13,
14. the solid pharmaceutical composition according to item 13, wherein the solid pharmaceutical composition is selected from the group consisting of a tablet, a granule, a capsules and a dry syrup.,
15. a method for manufacturing a fine particle-containing composition, the method comprising the steps of: (I) mixing a hardly-soluble drug, a surfactant and a poor solvent to obtain a liquid mixture; (II) pulverizing the liquid mixture in a wet disperser to obtain a fine particle dispersion; (III) adding a cyclic oligosaccharide to the fine particle dispersion; and (IV) drying the fine particle dispersion containing the cyclic oligosaccharide,
16. a method for manufacturing a fine particle-containing composition, the method comprising the steps of: (I) mixing a hardly-soluble drug, a surfactant, a poor solvent and a cyclic oligosaccharide to obtain a liquid mixture; (II) pulverizing the liquid mixture in a wet disperser to obtain a fine particle dispersion; and (III) drying the fine particle dispersion,
17. a method for manufacturing a fine particle-containing composition, the method comprising the steps of: (I) mixing a hardly-soluble drug, a surfactant and a first poor solvent to obtain a liquid mixture; (II) pulverizing the liquid mixture in a wet disperser to obtain a fine particle dispersion; (III) first drying the fine particle dispersion to obtain a first mixture; (IV) adding a cyclic oligosaccharide and a second poor solvent to the first mixture to obtain a second mixture; and (V) second drying the second mixture,
18. the method for manufacturing the fine particle-containing composition according to any one of items 15 to 17, wherein the hardly-soluble drug is mixed in the mixing step as a solution of the hardly-soluble drug dissolved in a good solvent,
19. the method for manufacturing the fine particle-containing composition according to any one of items 15 to 18, further comprising concentrating the fine particle dispersion or the fine particle dispersion containing the cyclic oligosaccharide prior to the drying step or the first drying step,
20. the method for manufacturing the fine particle-containing composition according to any one of items 15 to 19, wherein the drying step or the first drying step is a drying step employing a spray-drying,
21. the method for manufacturing the fine particle-containing composition according to any one of items 15 to 20, wherein the wet disperser is a homogenizer,
22. the method for manufacturing the fine particle-containing composition according to any one of items 15 to 21, wherein the surfactant is clathrated by the cyclic oligosaccharide in the addition step,
23. the method for manufacturing the fine particle-containing composition according to any one of items 15 to 22, wherein the cyclic oligosaccharide comprises a cyclodextrin,
24. the method for manufacturing the fine particle-containing composition according to any one of items 15 to 23, wherein the surfactant comprises a surfactant having a hydrocarbon chain with 4 or more carbon atoms,
25. the method for manufacturing the fine particle-containing composition according to any one of items 15 to 24, wherein an amount of the hardly-soluble drug is from 0.1 to 40 parts by weight based on 100 parts by weight of the fine particle-containing composition.

### Advantageous Effects of the Invention

According to the present invention, the aggregation and crystal growth of fine particles of a hardly-soluble drug over time are inhibited during the step of drying a suspension containing fine particles of the hardly-soluble drug and even in the dried composition through the additional use of a cyclic oligosaccharide in a technique of pulverizing the hardly-soluble drug by wet pulverization using a surfactant. The present invention also provides a fine particle-containing composition in which fine particles of the hardly-soluble drug are physically stable and are not affected by environmental conditions such as temperature and humidity during storage. Consequently, according to the present invention, fine particles of the hardly-soluble drug can be easily mixed into tablets and capsules, which are widely used as oral preparations, as well as into dry syrups and the like which can be re-dispersed in water and prepared as needed. Moreover, according to the present invention, the hardly-soluble drug can be orally administered with the size of the fine particles maintained, promoting absorption of the hardly-soluble drug, enhancing bioavailability, or allowing the dosage of the drug to be reduced, thereby providing a drug composition with excellent compliance. In addition, because the fine particle-containing composition of the present invention or a drug composition containing it has excellent storage stability, it can be made widely available because it is easy to transport and distribute as a pharmaceutical premix material or as a pharmaceutical product.

### Brief Description of Drawings

Figure 1 is a flow chart of manufacturing steps illustrating the first embodiment of the present invention.
Figure 2 is a flow chart of manufacturing steps illustrating the second embodiment of the present invention.
Figure 3 is a flow chart of manufacturing steps illustrating the third embodiment of the present invention.
Figure 4 is a flow chart of manufacturing steps illustrating the fourth embodiment of the present invention.
Figure 5 shows changes in average particle size of fine particles of glibenclamide fine particle-containing dispersions versus α-CD / SDS mole ratio.
Figure 6 shows solubility of glibenclamide in the presence of both α-CD and SDS.
Figure 7 shows the dissolution profiles of tablets containing fine particles of glibenclamide.
Figure 8 shows the dissolution profile of a tablet containing fine particles of glibenclamide before and after a storage test.

### Best Mode for Carrying Out the Invention

The following embodiments are exemplary for explaining the present invention, and do not intend to limit the present invention to these embodiments. The present invention can be implemented in a variety of modes as long as there is no departure from the spirit and the scope of the present invention.

The fine particle-containing composition according to the present invention is a composition containing fine particles of a hardly-soluble drug in a solid phase. The fine particle-containing composition according to the present invention comprises a hardly-soluble drug, a surfactant and a cyclic oligosaccharide, with fine particles of the hardly-soluble drug dispersed in the composition. This composition may also comprise other sugars, polymer substances and other additives as necessary. The fine particle-containing composition according to the present invention can be obtained by further adding a cyclic oligosaccharide to a fine particle dispersion containing fine particles of the hardly-soluble drug that have been wet pulverized in a liquid containing the hardly-soluble drug and the surfactant, and then drying the dispersion. The cyclic oligosaccharide may also be mixed with the hardly-soluble drug and surfactant before wet pulverization. The fine particle-containing composition can also be obtained by drying the dispersion of the fine particles of the hardly-soluble drug that have been wet pulverized in a solution containing the hardly-soluble drug and the surfactant, and then adding and mixing a poor solvent and a small quantity of a cyclic oligosaccharide into the resulting composition containing fine particles of the hardly-soluble drug and the surfactant, and drying the result. In these manufacturing methods, the cyclic oligosaccharide forms a clathrate with some or all of the surfactant, thereby contributing to the physical and chemical stability of the fine particles of the hardly-soluble drug during the drying step and during storage of the fine particle-containing composition.

The fine particles of the present invention are so-called nanoparticles, with an average particle size of 1 µm or less, and this average particle size can be measured by the light-scattering method. The composition containing fine particles of the hardly-soluble drug can be mixed with a poor solvent to disperse the fine particles of the hardly-soluble drug, and after dilution as necessary, the particles can be measured with a device capable of measuring nanometer-sized particles, such as an Otsuka Electronics DLS-7000 light scattering spectrophotometer or ELS-8000 laser zeta electrometer. Similarly, the particles of the hardly-soluble drug in the fine particle dispersion can be measured after being diluted as necessary with a poor solvent for the hardly-soluble drug. When the average particle size of the fine particles of the hardly-soluble drug in the present invention is measured by the light-scattering method with the solid dispersion dispersed in water, the upper limit of the average particle size is 1000 nm, preferably 900 nm, more preferably 800 nm. The lower limit of the average particle size is not particularly limited, but may be 50 nm, preferably 100 nm, more preferably 200 nm. Consequently, the average particle size of the fine particles of the hardly-soluble drug in the present invention is from 50 nm to 1000 nm, preferably from 100 nm to 900 nm, more preferably from 200 nm to 800 nm.

In the present invention, the term "drug" refers to therapeutic drugs and diagnostic drugs. The therapeutic drug may be a synthetic compound or a biological drug such as a protein or peptide, while the diagnostic drug may be an X-ray contrast agent or other drug used in diagnosis. The drug is preferably one that is hardly-soluble and can be dispersed in at least one kind of liquid solvent. The term "hardly-soluble" as used herein means that the drug is "slightly soluble" or "very slightly soluble" or "practically insoluble" in the solvent for that drug as defined in the 14^{th} Japanese Pharmacopoeia (hereinafter referred to as "Japanese Pharmacopoeia") or the 24^{th} U.S. Pharmacopoeia (hereinafter referred to as "USP"). Specifically, according to the Japanese Pharmacopoeia, solubility is a degree to which the drug in a solid form dissolves within 30 minutes after powdered, placed in the solvent, and strongly agitated for 30 seconds every 5 minutes at 20±5°C, and the term "slightly soluble" means that at least 100 mL but less than 1000 mL of water is required to dissolve 1 g of the drug. The term "very slightly soluble" means that at least 1000 mL but less than 10,000 mL of water are required to dissolve 1 g of the drug, while the term "practically insoluble" means that 10,000 mL or more of water are required to dissolve 1 g of the drug. In the present invention, the hardly-soluble drug is preferably a drug that is "practically insoluble" or "very slightly soluble" in water, or in other words a drug that requires at least 1000 mL of water to dissolve 1 g of the drug. Preferably, the hardly-soluble drug is a drug that is "practically insoluble", or in other words a drug that requires at least 10,000 mL of water to dissolve 1 g of the drug.

The hardly-soluble drug of the present invention is not particularly limited, but, for example, can be selected from a variety of drugs, including steroid agents, enzyme-inhibiting agents, analgesics, anti-fungal agents, cancer treatment agents, antiemetics, analgesics, circulatory agents, antiinflammatory agents, anti-parasitic agents, anti-arrhythmic agents, antibiotics (including penicillin), anticoagulants, anti-depressants, anti-diabetic agents, anti-epileptic agents, anti-dementia agents, antihistamines, anti-hypertensives, antimuscarinic agents, anti-mycobacterial agents, anti-malignant tumor agents, immunosuppressants, anti-thyroid agents, antiviral agents, anti-anxiety analgesics (sleeping agents and neuroleptics), astringents, β -adrenaline receptor antagonists, blood preparations and substitutes, heart stimulants, contrast agents, corticosteroids, cough suppressants (expectorants and mucolytic agents), diagnostic agents, diagnostic contrast agents, diuretics, dopaminergic agents (anti-Parkinson's agents), hemostatics, immune agents, lipid regulators, muscle relaxants, prostaglandins, radioactive drugs, hormones, anti-allergy agents, stimulants and appetite suppressants, sympathomimetics, thyroid agents, vasodilators and the like. One of these hardly-soluble drugs may be used or two or more may be used in combination.

An amount of the hardly-soluble drug in the fine particle-containing composition is from 0.1 to 40, preferably from 0.5 to 35, more preferably from 1 to 30, still more preferably from 1 to 25 parts by weight, based on 100 parts by weight of the fine particle-containing composition.

The surfactant of the present invention is used together with the hardly-soluble drug when pulverizing the hardly-soluble drug, and plays an important role in pulverizing the hardly-soluble drug. Alternatively, it helps to uniformly disperse the fine particles of the hardly-soluble drug when the fine particle-containing composition according to the present invention is re-dispersed in a solvent such as water. It is therefore not particularly limited as long as it has surfactant ability, but is preferably a high-HLB surfactant capable of improving the water dispersibility of the fine particles of the hardly-soluble drug, and examples of the surfactants include surfactants with an HLB of 8 or more, preferably 10 or more, more preferably 12 or more.

The surfactant of the present invention is also not particularly limited as long as part of the surfactant can be clathrated by cyclodextrin. Examples of the surfactants include surfactants having hydrocarbon chains that may be straight, branched or cyclic without any particular limitation, such as nonionic surfactants, ionic surfactants and natural surfactants having hydrocarbon chains. Preferably the surfactant of the present invention is one in which the hydrocarbon chain has 4 or more, preferably 6 or more, more preferably 8 or more carbon atoms, and still more preferably a surfactant in which the hydrocarbon chain has 10 or more carbon atoms. Specific examples of surfactants are given here but these examples are not limiting. Examples of nonionic surfactants include glycerin monostearate, sorbitan fatty acid ester, decaglyceryl monomyristate, hexaglyceryl monolaurate, glyceryl monooleate and other polyglycerin fatty acid esters (Nikko Chemicals), sucrose fatty acid ester (Mitsubishi-Kagaku Foods), polyoxyethylene castor oil derivatives (CO^{™} Series, Nikko Chemicals), polyoxyethylene hardened castor oil (for example, HCO^{™} Series, Nikko Chemicals), coconut oil mono-fatty acid polyoxyethylene sorbitan, polysorbate 80 (Tween 80 (product name), etc.), polysorbate 20 (Tween 20 (product name), etc.) and other polyoxyethylene sorbitan fatty acid esters, lauromacrogol, polyoxyethylene (20) cetyl ether, polyoxyethylene (15) oleyl ether and other polyoxyethylene alkyl ethers (Nikko Chemicals), polyoxyethylene (20) polyoxypropylene (4) cetyl ether and other polyoxyethylene polyoxypropylene alkyl ethers (Nikko Chemicals), and polyethylene glycol monostearate, polyethylene glycol distearate, polyethylene glycol monooleate and other polyethylene glycol fatty acid esters (Nikko Chemicals) and the like. Examples of ionic surfactants include fatty acid soaps, sodium stearoyl lactate, calcium stearoyl lactate and other acyl lactic acid salts, sodium dodecyl sulfate (Wako Pure Chemical Industries) and other alkyl sulfuric ester salts, alkyl phosphoric acid salts, benzalkonium chloride, cetyl pyridinium chloride and the like. Examples of natural surfactants include soy lecithin (True Lecithin Industries), hydrogenated soy phospholipids (True Lecithin Industries), egg yolk lecithin (Q.P. Corporation), lysolecithin (Kyowa Hakko Kogyo), lecithin hydroxide (Nikko Chemicals), and other lecithins and sodium cholate, sodium deoxycholate and other cholic acid salts. One such surfactant may be used, or two or more may be used in combination. An alkyl sulfuric ester salt, sucrose fatty acid ester, polyoxyethylene sorbitan fatty acid ester or polyglycerin fatty acid ester is preferred, and an alkyl sulfuric ester salt or sucrose fatty acid ester or polysorbate 80 or polysorbate 20 is most preferred.

The cyclic oligosaccharide used in the present invention is not particularly limited as long as it has the function of clathrating the surfactant, and examples include cyclic oligosaccharides having of 4 to 12 glucose units and cyclic lacto-oligosaccharides having lactic acid units, with α -cyclodextrin (Nihon Shokuhin Kako), β -cyclodextrin (Nihon Shokuhin Kako), γ-cyclodextrin (Nihon Shokuhin Kako) or a cyclic tetrasaccharide (Hayashibara Biochemical Laboratories) being preferred, and α-cyclodextrin, β-cyclodextrin or γ-cyclodextrin being especially preferred. One such cyclic oligosaccharide may be used, or two or more may be used in combination.

The relative amounts of the hardly-soluble drug, the surfactant and cyclic oligosaccharide in the fine particle-containing composition according to the present invention are not particularly limited, and other components may also be included. For example, the mixture proportion of the cyclic oligosaccharide based on 1 part by weight of the hardly-soluble drug in the fine particle-containing composition is from 0.5 to 1000, preferably from 1 to 500, more preferably from 2 to 200, still more preferably from 3 to 100 parts by weight. The mixture proportion of the surfactant based on 1 part by weight of the hardly-soluble drug in the fine particle-containing composition is from 0.1 to 10, preferably from 0.2 to 5, more preferably from 0.2 to 2, still more preferably from 0.4 to 1 parts by weight.

The method for manufacturing the fine particle-containing composition according to the present invention comprises a pulverization step in which the hardly-soluble drug is pulverized in the presence of a surfactant, an addition step in which a cyclic oligosaccharide is added, and a drying step in which fine particle dispersion is dried. The surfactant that was used in the pulverization step (specifically free surfactant and surfactant adhering to the surface of the hardly-soluble drug) is clathrated due to the inclusion of an addition step in which a cyclic oligosaccharide is added. As a result, aggregation of fine particles of the hardly-soluble drug and crystal growth of the hardly-soluble drug due to the presence of the surfactant are suppressed during the drying step or in the dried composition. Consequently, the following embodiments can be given as examples of the manufacturing method of the present invention.

### (First Embodiment of the Present Invention)

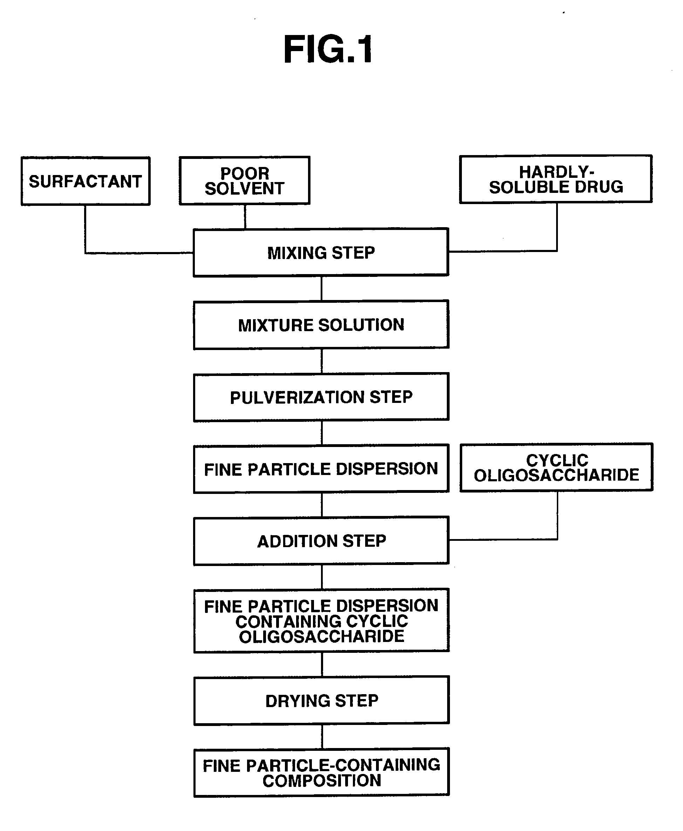

Figure 1 illustrates steps of the first embodiment with respect to the method for manufacturing the fine particle-containing composition according to the present invention. In this embodiment, the hardly-soluble drug is first pulverized, and the cyclic oligosaccharide is then added to the fine particle dispersion. That is, the fine particle-containing composition according to the present invention can be obtained by first (I) mixing the hardly-soluble drug, the surfactant and a poor solvent to obtain a mixture in the mixing step. Next, (II) this mixture is pulverized in a wet disperser to obtain a fine particle dispersion in the pulverization step. Further, (III) the cyclic oligosaccharide is added to and mixed with the fine particle dispersion in the addition step, after which (IV) this fine particle dispersion containing the cyclic oligosaccharide is dried in the first drying step to obtain the fine particle-containing composition according to the present invention.

### (Second Embodiment of the Present Invention)

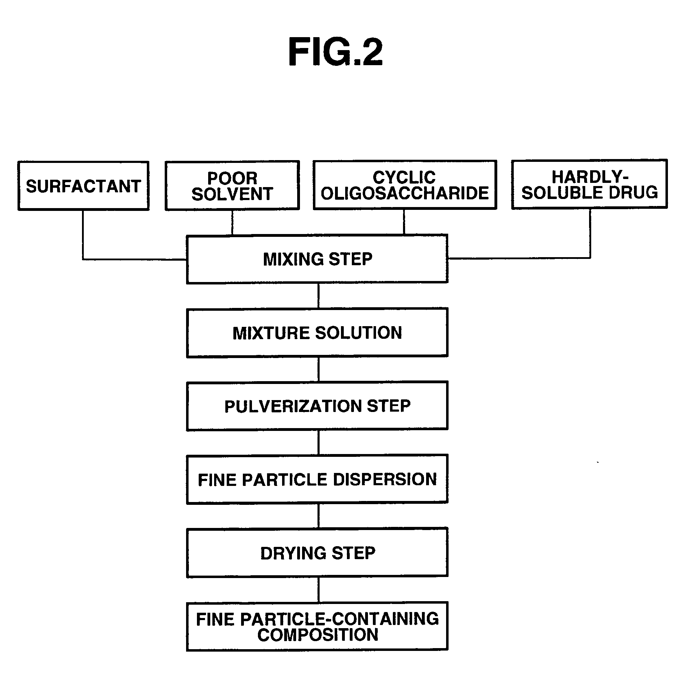

Figure 2 illustrates steps of the second embodiment with respect to the method for manufacturing the fine particle-containing composition according to the present invention. The feature of this embodiment is that the hardly-soluble drug is refined in the presence of the cyclic oligosaccharide. That is, (I) the hardly-soluble drug, the surfactant, the cyclic oligosaccharide and a poor solvent are mixed in the mixing step to obtain a liquid mixture. Next, (II) the liquid mixture containing the cyclic oligosaccharide is pulverized in a wet disperser in the pulverization step to obtain a dispersion of fine particles of the hardly-soluble drug. Finally, (III), the fine particle dispersion containing the cyclic oligosaccharide is dried in the drying step to obtain the fine particle-containing composition according to the present invention.

### (Third Embodiment of the Present Invention)

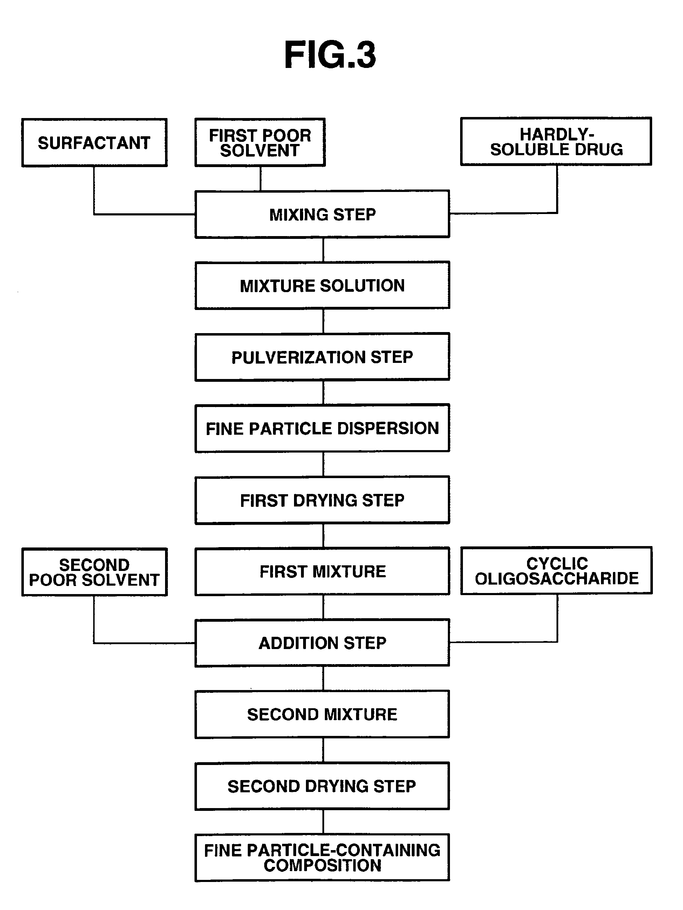

Figure 3 illustrates steps of the third embodiment with respect to the method for manufacturing the fine particle-containing composition according to the present invention. The feature of this embodiment is that a composition containing a surfactant and fine particles of the hardly-soluble drug is first obtained, and the cyclic oligosaccharide and a fine solvent are then added thereto and mixed. That is, (I) the hardly-soluble drug, the surfactant and a first poor solvent are first mixed in the mixing step to obtain a liquid mixture. Next, (II) this liquid mixture is pulverized in a wet disperser in the pulverization step to obtain a fine particle dispersion. Further, (III) this fine particle dispersion is dried in the first drying step to obtain a mixture (hereinafter referred to as "first mixture") containing the surfactant and fine particles of the hardly-soluble drug. Next, (IV) the cyclic oligosaccharide and a second poor solvent are added to the first mixture in the addition step and mixed to obtain a mixture (hereinafter referred to as "second mixture"). Finally, (V) the second mixture is dried in the second drying step in order to remove the second poor solvent, thereby obtaining a fine particle-containing composition. The first and second poor solvents may be either the same or different. Another sugar or polymer may also be used in the step of obtaining the first mixture or the second mixture. There are no particular limits on the method used in the second drying step, which may be any capable of removing the second solvent.

### (Fourth Embodiment of Invention)

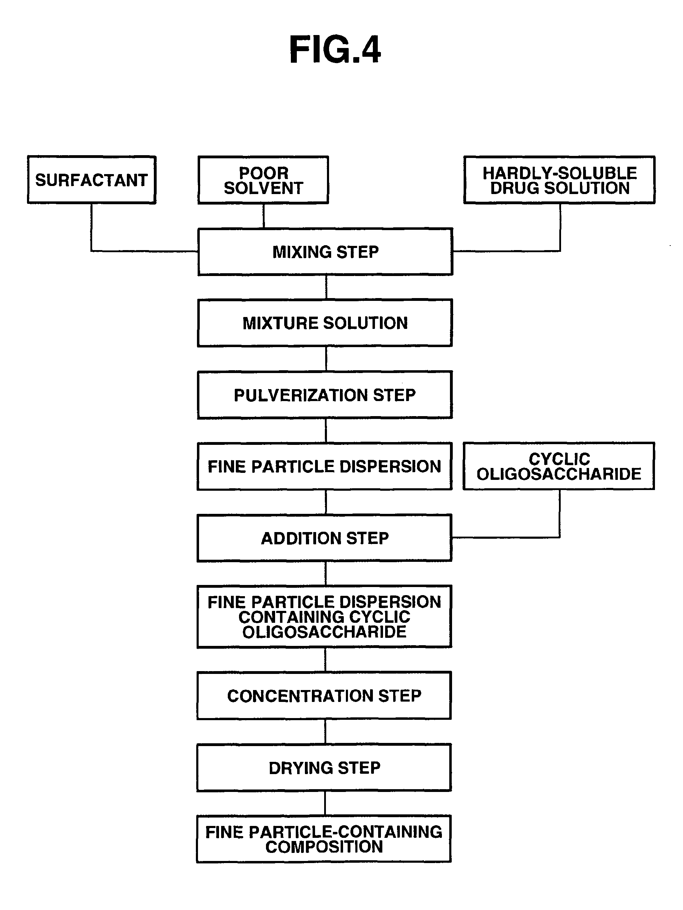

The pulverization step in this invention employs dispersion technology using a homogenizer, mill or other wet disperser and a surfactant, but preferably this can also be combined with crystallization technology. This can be combined with the first embodiment for example as shown in Figure 4. That is, the hardly-soluble drug in the form of a solution of the hardly-soluble drug dissolved in a good solvent is mixed with the surfactant and the poor solvent to obtain a liquid mixture in the mixing step, after which this mixture is pulverized in a wet disperser to obtain a fine particle dispersion in the pulverization step. In this case, the surfactant or cyclic oligosaccharide is preferably mixed by in advance dissolving or dispersing it in the solution of the hardly-soluble drug or in the poor solvent. A concentration step is preferably included before the drying step in order to remove the good solvent from the liquid mixture or to remove both the good solvent and the poor solvent. Specifically, the fine particle dispersion containing the cyclic oligosaccharide can be concentrated by removing the good solvent and poor solvent by ultrafiltration or dialysis. Then, the fine particle-containing composition according to the present invention can be obtained by means of a drying step. Finer particles can also be obtained and the effects of the present invention enhanced by including such a crystallization technique and concentration step in the second and third embodiments as well as in the first embodiment.

The first, second or fourth embodiment can also be combined with the third embodiment in the method for manufacturing the fine particle-containing composition according to the present invention. For example, an addition step of adding and mixing cyclic oligosaccharide and the second poor solvent and the second drying step to remove the second poor solvent can be added after the fine particle-containing composition according to the present invention has been obtained by means of the first embodiment. Alternatively, it is possible to include an addition step of adding and mixing cyclic oligosaccharide and the poor solvent into a composition containing the surfactant and fine particles of the hardly-soluble drug obtained by a different method, followed by a drying step to remove the added poor solvent. However, the embodiments of the present invention are not limited to the foregoing.

The mixing step of the present invention is a step in which, prior to the pulverization step of the present invention, the hardly-soluble drug, the surfactant and poor solvent are mixed, along with cyclic oligosaccharide and other additives as necessary, to obtain a liquid mixture. In order to obtain fine particles efficiently in the subsequent pulverization step, the various components should preferably be mixed uniformly in the liquid mixture. The mixing step can therefore include various steps for dispersing the particles of the hardly-soluble drug, crushing the drug powder and modifying the surface of the drug powder. For example, the wet disperser used in the pulverization step can be used as necessary in addition to a propeller agitator, Homo Disper (Mizuho Industrial), Homo Mixer (Mizuho Industrial), Homo Jettor (Tokushu Kika Kogyo) pressure emulsifier, colloid mill (Tokushu Kika Kogyo), jet mill (Kurimoto), crusher or the like during the operations of agitating the liquid mixture, pulverizing the drug powder and the like. For example, the hardly-soluble drug can be roughly pulverized as is in the jet mill or other mill and then mixed with the surfactant, poor solvent and the like in the Homo Mixer. Alternatively, the hardly-soluble drug and surfactant can be kneaded or mixed in the mill, the grinder or the like, and then pulverized in the wet disperser of the present invention after addition of a solvent.

The surfactant is used as a means for dispersing the hardly-soluble drug in the particle pulverization step of the present invention, in combination with a wet disperser. That is, in this step the hardly-soluble drug is pulverized with the wet disperser in the liquid mixture obtained by the mixing step of the present invention, in other words, in a solution containing the hardly-soluble drug and the surfactant in order to obtain a fine particle dispersion containing fine particles of the hardly-soluble drug. The concentration of the surfactant in the liquid mixture during pulverization is not particularly limited, but is generally from 0.01 to 5 w/v%, preferably from 0.1 to 4 w/v%, more preferably from 0.3 to 3 w/v%.

When crystallization technology is used in the pulverization step of the present invention, fine particles of the hardly-soluble drug can be precipitated by means of a mixing step in which a solution of the hardly-soluble drug dissolved in the good solvent is mixed with the poor solvent in the mixing step of the present invention. In this case, the surfactant used in the mixing step can be added to either the poor solvent or the good solvent, or to both. Moreover, when using crystallization technology the wet disperser is preferably used immediately after the mixing step. That is, a fine particle dispersion of the hardly-soluble drug is prepared by pulverization in the wet disperser within generally 5 minutes, preferably 3 minutes, more preferably 1 minute after the mixing step.

The good solvent used in the present invention is a solvent that completely dissolves the hardly-soluble drug, without any particular limitations, but examples of the good solvent include methyl alcohol, ethyl alcohol, n-propyl alcohol, isopropyl alcohol and other lower alcohols, acetone, methyl ethyl ketone and other ketones, and acetonitrile, dioxane, methyl ether, chloroform and mixed solvents of these. The poor solvent of the present invention is one that dissolves the hardly-soluble drug very little, without any particular limitations, but examples of the poor solvent include water, acidic water with various acids added, and basic water with various bases added. When using crystallization technology, the poor solvent is preferably a solvent that mixes with the hardly-soluble drug dissolved in the good solvent. When the hardly-soluble drug dissolved in the good solvent is mixed together with the poor solvent, the mixing ratios thereof are not particularly limited as long as they induce precipitation of the drug, but the amount of the good solvent is generally from 0.001 to 50 v/v%, preferably from 0.01 to 10 v/v%, more preferably from 0.01 to 5 v/v% based on the amount of the poor solvent.

The wet disperser used in the pulverization step of the present invention is not particularly limited as long as it is capable of making the hardly-soluble drug into the fine particles of the present invention. Mills that can be used include those using rotating compression by means of beads, balls, rings or rollers, and those using a shearing effect. Specific examples include Dyno-mill (W. A. Bachofen, Switzerland), Ball Mill (Fritsch, Germany), Micros (Nara Machinery), Super Clean Mill (Nara Machinery), Drais Bead Mill (Draiswerke, U.S.) .and the like. The mechanism of the homogenizer is not particularly limited as long as it is capable of achieving shearing force, shock force, cavitation force, high-speed flow or ultrasound sufficient to produced fine particles of the hardly-soluble drug. Examples of the homogenizer include high-pressure homogenizers that collide process liquids with each other or force them through fine orifices, high-speed rotational homogenizers that employ cavitation or shearing force caused by micro-clearance using rotors, stators, screens or the like, and other homogenizers such as ultrasound homogenizers. Specific examples of high-pressure homogenizers, but are not limited to, include Nanomizer (Yoshida Kikai), Microfluidizer (MFI, U.S.), piston gap homogenizer (EmulsiFlex-C160, Avestin, Canada), APV homogenizer (Invensys Systems), Clear-SS5 (M. Technique), Niro Soavi Homogenizer (Doyei), Ultimizer (Sugino Machine) and the like. Specific examples of high-speed rotational homogenizers include the high-performance dispersion emulsifier (Clearmix®, M. Technique) Polytron® homogenizer (Kinematica), Hiscotron® (Microtech Nichion) and the like. Examples of ultrasound homogenizers include the high-efficiency ultrasound homogenizer (Nihon SiberHegner) and the like. The wet disperser of the present invention is preferably a homogenizer and more preferably a high-pressure homogenizer.

When a high-pressure homogenizer is used as the wet disperser of the present invention, the pressure during pulverization depends on the machine capability and is not particularly limited, but when other dispersion technology is not used it is generally from 14,000 to 60,000 psi, preferably from 20,000 to 60,000 psi, more preferably from 30,000 to 60,000 psi. When crystallization technology or the like is also used, the pressure during pulverization in the high-pressure homogenizer may be surprising low - generally from 500 to 40,000 psi, preferably from 1000 to 30,000 psi, more preferably from 3000 to 30,000 psi. The temperature of the liquid during pulverization with the high-pressure homogenizer in the present invention is not particularly limited, but is generally a temperature at which the hardly-soluble drug does not completely dissolve in the solvent, with the lower limit being the temperature at which the solvent does not solidify or become viscous, and specifically the temperature is from 1 to 40°C, preferably from1 to 30°C. Moreover, in pulverization with a high-pressure homogenizer the number of passes is not particularly limited, and the target fine particles can be obtained with an in-line continuous operation.

When a high-speed rotational homogenizer is used as the wet disperser of the present invention, the rotational speed during pulverization depends on the machine, but is generally at least 12,000 rpm, preferably at least 15,000 rpm, more preferably at least 18,000 rpm. The temperature of the liquid being processed during this process is not particularly limited, but is generally a temperature at which the hardly-soluble drug does not completely dissolve in the solvent, with the lower limit being the temperature at which the solvent does not solidify or become viscous, and specifically the temperature is from 1 to 40°C, preferably from 1 to 30°C.

When the mill is used as the wet disperser of the present invention, the method of preparing the fine particle dispersion comprises: first rough-treating the hardly-soluble drug in a solution containing a surfactant; and then abrading it with a grinding medium.

The addition step of the present invention is a step in which cyclic oligosaccharide is added with the aim of clathrating the surfactant with the cyclic oligosaccharide. The cyclic oligosaccharide may be added after having been dissolved or dispersed as necessary in a poor solvent for the hardly-soluble drug, such as water. There are not particular limits on the mixing method as long as the surfactant and cyclic oligosaccharide are brought into contact during the mixing process. For example, agitation operations and equipment can be used in the mixing process. When the cyclic oligosaccharide is added to the first mixture containing the surfactant and fine particles of the hardly-soluble drug as in the third embodiment, the second poor solvent is also added and mixed. The second poor solvent may be the same as or different from the first poor solvent used in the mixing step. There are no particular limits on the method of adding and mixing the second poor solvent or on the added amount of the second poor solvent, and the second mixture obtained by adding the second poor solvent may be a liquid, a paste, or a wet powder. Preferably, in the addition step of the third embodiment the second poor solvent can be added together with the cyclic oligosaccharide as a binder liquid for wet granulation to the first mixture obtained by the first drying step. The fine particle-containing composition according to the present invention can be obtained by drying this granulated product.

The drying step of the present invention is a step of drying the fine particle dispersion or the second mixture, thereby eliminating the good solvent or poor solvent contained therein. Drying can be carried out by the known methods such as spray-drying, fluidized bed granulation, freeze drying, tray drying or the like, and either one method or a combination of multiple methods may be used, but these examples are not limiting. In the case of spray-drying, a fine particle-containing composition can be obtained by spray-drying the fine particle dispersion of the present invention with a spray dryer or the like. The inflow air temperature during spray-drying is generally from 80°C to 200°C or more, preferably from 90°C to 180°C, more preferably from 100°C to 160°C. The solids concentration of the fine particle dispersion during spray-drying is, for example, from 0.5 to 30%, preferably from 1 to 20%, more preferably from 3 to 15%. The solids concentration herein indicates the component in the dispersion that does not volatilize during the drying step, or in other words the total concentration of the hardly-soluble drug, the surfactant, cyclic oligosaccharide, and other excipients and the like. In the case of fluidized bed granulation, a fine particle-containing composition is obtained by spraying and granulating the fine particle dispersion of the present invention on a lactose, starch or other powder. In the case of freeze drying, the fine particle-containing composition is obtained by adding a sugar or other tonicity agent to the fine particle dispersion, and then using a freeze dryer to remove the solvent by freezing at generally from -20°C to -60°C. In the case of tray drying, a fine particle-containing composition is obtained by drying the fine particle dispersion at generally from 50°C to 80°C, either as is or after adsorption of the solvent with an excipient. The drying step of the present invention (or the first drying step in the third embodiment) preferably employs spray-drying, freeze drying or fluidized bed drying, and spray-drying is particularly preferred. Spray-drying may be followed by secondary drying using a fluidized bed apparatus or tray dryer. In the second drying step of the third embodiment, the drying method may be selected according to the added amount of the second poor solvent, in other words according to the nature of the second mixture. For example, if the second mixture is a liquid the drying method can be similar to that used to dry the fine particle dispersion. If it is a wet powder, fluidized bed granulation may be used. The drying step of the present invention may also include adsorption with anhydrous silicate or the like, and solidification may also be accomplished by other methods.

Excipients, binders and other additives required for pulverization that are used in the known methods may be mixed in the drying step of the present invention. Since the cyclic oligosaccharide is added to physically stabilize the fine particles of the hardly-soluble drug, however, such additives should not be added more than necessary. Thus, the resulting composition can be applied to various types of solid pharmaceutical compositions because it is mixed with a higher content of finer particles of the hardly-soluble drug than the hardly-soluble drug concentration of the final pharmaceutical product.

The fine particle-containing composition of the present invention can be administered orally, rectally, parenterally (intravenously, intramusucularly, subcutaneously), intracapsularly, transvaginally, intraperitoneally, locally, intraorally or nasally to humans or animals.

The pharmaceutical compositions for various kinds of administration can also be formulated using the fine particle-containing composition according to the present invention. The pharmaceutical composition according to the present invention can be administered orally in the form of tablets, powders, fine-granules, granules, capsules, pills, dry syrups or troches, or as liquids, suspensions, emulsions, elixirs or syrups, troches or may be administered parenterally in the form of an inhalants, suppositories, injections, ointments, skin patches, cataplasms, ophthalmic ointments, ophthalmic drops, nosal drops, ear drops, lotion or the like. In this case, two or more kinds of fine particle-containing compositions containing different drugs or concentrations can be used.

The fine particle-containing composition may be used as is as the pharmaceutical composition according to the present invention, but pharmacologically acceptable additives may also be used. In the case of a solid pharmaceutical composition, commonly-used excipients, binders, disintegrators, lubricants, colorants, coatings and the like may be used. These additives may be mixed and the solid pharmaceutical composition prepared by a combination of the known techniques such as mixing, granulation, compression, tableting, capsule filling and the like.

An injection can be prepared by the ordinary methods with solubilizers, tonicity agents, stabilizers, analgesics, buffers, suspending agents, anti-oxidants and the like added as necessary. An injection may be a freeze-dried preparation. Because an injection must be sterile, it should be high-pressure heat sterilized for 8 minutes or more at 121°C for example. An injection may be administered intravenously, intracutaneously, subcutaneously or intramuscularly. The injection of the present invention is preferably one that is prepared at the time of use. The fine particle-containing composition may be used as is as an injection that is prepared at the time of use, but sucrose, glucose, D-mannitol, sodium chloride and other isotonic agents may also be added thereto. For example, these tonicity agents may be added as necessary to the fine particle dispersion of the hardly-soluble drug, which can then be freeze dried to obtain a fine particle-containing composition which is used as an injection that is prepared at the time of use.

The external preparation of the present invention can be manufactured by the ordinary methods as an ointment, cream, suppository, patch, lotion or the like, with bases, emulsifiers, thickeners, preservatives, stabilizers and the like added to the fine particle-containing composition according to the present invention. Like an injection, it can also be manufactured as a lotion that is prepared at the time of use. Various raw materials that are generally used in drugs, quasi-drugs, cosmetics and the like can be used as base materials, such as animal or vegetable fats, mineral oils, ester oils, waxes, higher alcohols, fatty acids, silicon oil, surfactants, phospholipids, alcohols, polyvalent alcohols, water-soluble polymers, clay minerals, purified water and other raw materials, and pH adjusters, anti-oxidants, chelating agents, preservatives, colorants, perfumes and the like can also be added as necessary.

Specific examples of additives that can be used in the drug composition of the present invention include, but are not limited to, excipients such as D-mannitol, lactose (including lactose anhydride), sucrose (including purified sucrose), sodium bicarbonate, corn starch, potato starch, wheat starch, rice starch, partial alpha starch, crystal cellulose, light silicic anhydride, calcium hydrogenphosphate anhydride, calcium hydrogen phosphate, tribasic calcium phosphate, precipitated calcium carbonate, calcium silicate and the like and binders such as povidone, dextrin, hydroxypropyl cellulose, hydroxypropyl methyl cellulose, methyl cellulose, polyvinyl alcohol, carboxymethyl cellulose sodium, alpha starch, sodium alginate, pullulan, gum arabic powder and the like. Examples of lubricants include, but are not limited to, hydrogenated oils, hydrogenated castor oil, stearic acid, magnesium stearate, calcium stearate, glyceride behenate, sodium stearyl fumarate and the like. Examples of disintegrators include, but are not limited to, low-substituted hydroxypropyl cellulose, carmellose, carboxymethyl starch sodium, crospovidone and the like. Examples of coatings include cellulose derivatives such as hydroxypropyl cellulose, hydroxypropyl methyl cellulose, ethyl cellulose, hydroxypropyl methyl cellulose phthalate, carboxymethyl ethyl cellulose, carmellose sodium, carmellose potassium, cellulose acetate, cellulose acetate phthalate and the like; acrylic acid-based polymers such as ethyl acetate-methyl methacrylate copolymer dispersion, aminoalkyl methacrylate copolymer E, aminoalkyl methacrylate copolymer RS, methacrylic acid copolymer L, methacrylic acid copolymer LD, methacrylic acid copolymer S, 2-methyl-5-vinylpyridine methyl acrylate-methacrylic acid copolymer, dimethylaminoethyl methacrylate-methyl methacrylate copolymer and the like; synthetic polymer substances such as polyvinylpyrrolidone, polyvinylacetal diethylaminoacetate, polyvinyl alcohol, polyoxyethylene polyoxypropylene glycol, macrogol and the like; polysaccharides such as pullulan, chitosan and the like; natural polymer substances such as gelatin, succinated gelatin, gum arabic, shellac and the like. Examples of plasticizers include, but are not limited to, dioctyl adipate, triethyl citrate, triacetin, glycerin, concentrated glycerin, propylene glycol and the like. Examples of suspending agents or emulsifiers include, but are not limited to, lecithin, sucrose fatty acid esters, polyglycerin fatty acid esters, polyoxyethylene hydrogenated castor oil, polysorbate, polyoxyethylene-polyoxypropylene copolymer and the like. Examples of flavorings include, but are not limited to, menthol, cinnamon oil, lemon oil, orange oil and the like. Examples of anti-oxidants include, but are not limited to, sodium ascorbate, L-cysteine, sodium sulfite, natural vitamin E and the like. Examples of sugar coatings include, but are not limited to, sucrose, lactose, starch syrup, precipitated calcium carbonate, gum arabic, carnauba wax, shellac, beeswax, macrogol, ethyl cellulose, methyl cellulose, povidone and the like. Examples of moisture-proofing agents include, but are not limited to, magnesium silicate, light silicic anhydride, hydrogenated oil, stearic acid, magnesium stearate, paraffin, castor oil, macrogol, vinyl acetate resin, cellulose acetate phthalate, polyvinylacetal diethylaminoacetate, shellac and the like. Examples of fluidizers include, but are not limited to, silicon dioxide hydrate, light silicic anhydride, heavy silicic anhydride, crystalline cellulose, synthetic aluminum silicate, alumina magnesium hydroxide, magnesium metasilicate aluminate, stearic acid, calcium stearate, magnesium stearate, calcium triphosphate, talc, corn starch and the like. Examples of colorants include, but are not limited to, yellow food color #4, yellow food color #5, red food color #2, red food color #102, blue food color #1, blue food color #2 (indigo carmine), yellow #4 aluminum lake and other tar dyes, yellow iron sesquioxide, iron sesquioxide (red iron oxide), black iron oxide, titanium oxide, zinc oxide, talc, curcuma extract, caramel, carotene, beta-carotene, copper chlorophyll, sodium copper chlorophyll, riboflavin, carbon black, pharmaceutical carbon and the like. Examples of solvents include, but are not limited to, water, ethyl alcohol, isopropyl alcohol, ethyl carbonate, ethyl acetate, benzyl alcohol, benzyl benzoate, propylene glycol, 1,3-butylene glycol, dimethylformamide, cottonseed oil, peanut oil, corn germ oil, olive oil, castor oil, sesame seed oil, glycerin, polyethylene glycol and the like.

Specifically, the fine particle-containing composition according to the present invention can be prepared by the following methods. To an aqueous solution (38 mL) of sodium dodecyl sulfate (10.5 mg/mL) (Wako Pure Chemical, hereunder SDS) is added a dimethylsulfoxide (2 mL) (Wako Pure Chemical, hereinafter referred to as "DMSO") solution of glibenclamide (200 mg/mL, 4-[2-(5-Chloro-2-methoxybenzoylamino)ethyl](N-cyclohexylcarbamoyl)benzene-sulfonaide, being practically insoluble in water, (Wako Pure Chemical)), which is agitated, and then immediately transferred to a Nanomizer (Yoshida Kikai) and then subjected to Nanomizer treatment for 10 minutes, so as to obtain a fine particle dispersion. This dispersion is dialyzed with an aqueous SDS (10 mg/mL) solution to remove the DMSO and obtain a concentrated fine particle dispersion. Based on dispersion A (7.5 mL), α-Cyclodextrin (1350 mg) (Nihon Shokuhin Kako, hereinafter refeered to as "α-CD") and purified water (12.5 mL) are added thereto and then mixed. This solution is spray-dried with a Pulvis Mini-Spray GB22 spray dryer (Yamato Kagaku, hereinafter referred to as "S.D.-GB22") to obtain a solidified fine particle-containing composition. The fine particle-containing composition obtained in this way can be mixed with excipients, disintegrators, lubricants and the like and made into granules, tablets or capsules by granulation, tableting or capsule filling.

Although the present invention is explained in more detail below using examples, the present invention is not limited by these examples. The additives used in the pharmaceutical compositions are reagents or conform to the 2003 Japanese Standards for Pharmaceutical Additives, the 1997 Japanese Pharmaceutical Codex and other official standards.

### Examples

### (Example 1)

To an aqueous SDS (10.5 mg/mL) solution (38 mL) was added a DMSO solution (2 mL) of glibenclamide (200 mg/mL), which was stirred, and immediately transferred to a Nanomizer and then subjected to Nanomaizer treatment for 10 minutes, so as to obtain a fine particle dispersion. This dispersion was dialyzed with an aqueous SDS (10 mg/mL) solution to remove the DMSO and obtain a concentrated dispersion (hereinafter referred to as "dispersion A"). Based on dispersion A (7.5 mL), α-CD (1350 mg) and purified water (12.5 mL) were added thereto, and then mixed (solids concentration 7.5% = concentration of glibenclamide, SDS and α-CD in dispersion). This solution was spray-dried with an S.D.-GB22 at an inflow air temperature of 115°C to obtain a fine particle-containing composition (air flow rate 0.5 m³/min, atomizing air 1 kgf/cm², liquid supply rate 7 mL/min). The fine particle-containing composition was obtained by spray-drying. As a result, a fine particle-containing composition was obtained, which contained 5 parts by weight of glibenclamide, 5 parts by weight of SDS and 90 parts by weight of α-CD, based on 100 parts by weight of the fine particle-containing composition.

### (Examples 2 and 3)

β-cyclodextrin (Nihon Shokuhin Kako, hereinafter referred to as "β-CD") and γ-cyclodextrin (Nihon Shokuhin Kako, hereinafter referred to as "γ - CD") were used in place of α-CD in the manufacturing method of Example 1, so as to obtain, respectively, a fine particle-containing composition containing β -CD (Example 2) and a fine particle-containing composition containing γ -CD (Example 3).

### (Comparative Examples 1 to 6, Control Example 1)

Fine particle-containing compositions were obtained using the sugars shown in Table 1. The fine particle-containing composition containing lactose of Comparative Example 1 was prepared by the methods described in Example 1, with a spray-drying temperature of 175°C. The fine particle-containing composition containing D-mannitol of Comparative Example 2 was prepared by the methods described in Example 1. In the case of the fine particle-containing compositions containing erythritol, sucrose and trehalose, to an aqueous SDS (5.1 mg/mL) solution (39 mL) was added a DMSO solution (1 mL) of glibenclamide (200 mg/mL), which was stirred, and immediately transferred to a Nanomizer and then subjected to Nanomizer treatment for 10 minutes, so as to obtain a fine particle dispersion. This dispersion was dialyzed with an aqueous SDS (5 mg/mL) solution to remove the DMSO and obtain a concentrated dispersion (hereinafter referred to as "dispersion B"). Based on dispersion B (15 mL), each sugar (1350 mg) and purified water (5 mL) were added thereto, and then mixed (solids concentration 7.5%). This solution was spray-dried using an S.D.-GB22 at an inflow air temperature of 115°C (Comparative Example 3, Comparative Example 5, Control Example 1) or 175°C (Comparative Example 4) to obtain a fine particle-containing composition (air flow rate 0.5 m³/min, atomizing air 1 kgf/cm², liquid supply rate 7 mL/min). As a result, fine particle-containing compositions were obtained, which contained 5 parts by weight of glibenclamide, 5 parts by weight of SDS and 90 parts by weight of a sugar, based on 100 parts by weight of the fine particle-containing composition as in Example 1. Preparation was also attempted using sodium chloride in place of erythritol, but the fine particles aggregated or precipitated in the dispersion, and could not be spray-dried. In Control Example 1, no cyclodextrin or sugar was added and dispersion B (solids concentration 1 %) was spray-dried at 115°C (air flow rate 0.5 m³/min, atomizing air 1 kgf/cm², liquid supply rate 7 mL/min) to obtain a fine particle-containing composition containing no cyclodextrin (50 parts by weight of glibenclamide, 50 parts by weight of SDS, based on100 parts by weight of composition).]

### (Test Example 1)

### (Storage Test)

Each of the fine particle-containing compositions prepared in the above Examples and Comparative Examples was packed in a glass bottle without a lid and stored for 1 week in a storage room under environmental conditions of 60°C, 75% RH. The particle sizes of the fine particles in the various fine particle-containing compositions were measured before and after storage.

### (Method for Measuring Average Particle Size)

The fine particle-containing composition was added to purified water and stirred so as to obtain a solution of dispersed fine particles of the hardly-soluble drug. The glibenclamide concentration in the dispersion for measurement was set at 0.25 mg/mL. Particle size was measured by a dynamic light scattering using an ELS-8000 (Otsuka Electronics), and the cumulant diameter was taken as the average particle size. The difference between the particle size after storage and the initial particle size was calculated from the measured values.

The results are shown in Table 1. The fine particles of glibenclamide in dispersion A had an average particle size of 205.8 nm. In dispersion B, the average particle size was 198.6 nm. Surprisingly, in the fine particle-containing compositions containing cyclodextrin there was very little particle size change due to spray-drying, and the particle size of the fine particles of glibenclamide changed very little over time even when the pulverized product was stored at a high humidity.
With the sugars used as the control, after spray-drying the results were similar to those obtained with cyclodextrin, but after pulverization the average particle size increased, to 1.4 times or more even with D-mannitol, which exhibited the least change. In the control example using no cyclodextrin or sugar, the average particle size after spray-drying was 1000 nm or more. The average particle size also increased in the storage test. It has thus been shown that cyclic oligosaccharide helps to prevent aggregation of fine particles of the hardly-soluble drug and crystal growth over time both during spray-drying in the spray-drying step and during storage after manufacture. In particular, it has been shown to have the effect of dramatically suppressing particle size change of the solidified fine particles, or in other words, aggregation and crystal growth in a solidified state, much more than other sugars.

**[Table 1]**

| | Sugar | Average particle size (nm) | | Change (%) |
|---|---|---|---|---|
| | | Initial | After storage | |
| Example 1 | α-CD | 222.0 | 234.2 | 5% |
| Example 2 | β-CD | 243.4 | 221.5 | -9% |
| Example 3 | γ-CD | 224.8 | 231.0 | 3% |
| Comparative Example 1 | Lactose | 210.7 | 885.5 | 320% |
| Comparative Example 2 | D-mannitol | 219.8 | 310.5 | 41% |
| Comparative Example 3 | Erythritol | 218.8 | 2230.1 | 919% |
| Comparative Example 4 | Sucrose | 209.3 | 1224.2 | 485% |
| Comparative Example 5 | Trehalose | 203.6 | 874.3 | 329% |
| Comparative Example 6 | Sodium chloride | Aggregation · precipitation | | - |
| Control Example 1 | Not added | 1257.6 | 3915.4 | 211% |

| | | | | |
|---|---|---|---|---|
| Mixture ratio: glibenclamide/SDS/sugar = 5/5/90 Control Example 1: glibenclamide/SDS/sugar = 50/50/0 | | | | |

### (Example 4)

Dispersion A was prepared by the method described in Example 1. Based on this dispersion A (7.5 mL), α-CD (225 mg) was added thereto, and then mixed (solids concentration 5%). This solution was spray-dried using an S.D.-GB22 at an inflow air temperature of 115°C to obtain a fine particle-containing composition (air flow rate 0.5 m³/min, atomizing air 1 kgf/cm², liquid supply rate 7 mL/min). As a result, a fine particle-containing composition was obtained, which contained 20 parts by weight of glibenclamide, 20 parts by weight of SDS and 60 parts by weight of α-CD, based on 100 parts by weight of the fine particle-containing composition.

### (Comparative Example 7)

D-mannitol was used in place of α-CD in the method of Example 4. D-mannitol suppressed changes in average particle size of the fine particles more than any of the sugars other than CD in Test Example 1.

### (Test Example 2)

A storage test was performed and the average particle size of the fine particles of the hardly-soluble drug was measured as in Test Example 1. The results are shown in Table 2. Even in a solidified fine particle-containing composition containing a high concentration of the drug, cyclic oligosaccharide suppressed changes in particle size due to aggregation and crystal growth over time. The average particle size of the fine particles in dispersion A was 214.8 nm in Example 4.

**[Table 2]**

| | Sugar | Average particle size (nm) | | Change (%) |
|---|---|---|---|---|
| | | Initial | After storage | |
| Example 4 | α-CD | 251.8 | 524.4 | 108% |
| Comparative Example 7 | D-mannitol | 233.6 | 1095.5 | 369% |

| | | | | |
|---|---|---|---|---|
| Mixture ratio: glibenclamide/SDS/sugar = 20/20/60 | | | | |

### (Example 5)

Based on dispersion A (7.5 mL) described in Example 4, lactose (1275 mg), α-CD (75 mg) and purified water (12.5 mL) were added thereto, and then mixed (solids concentration 7.5%). This solution was spray-dried using an S.D.-GB22 with an inflow air temperature of 175°C to obtain a solidified fine particle-containing composition (air flow rate 0.5 m³/min, atomizing air 1 kgf/cm², liquid supply rate 7 mL/min). As a result, a fine particle-containing composition was obtained, which contained 5 parts by weight of glibenclamide, 5 parts by weight of SDS, 5 parts by weight of α-CD and 85 parts by weight of lactose, based on 100 parts by weight of the fine particle-containing composition.

### (Example 6)

Based on dispersion A (7.5 mL) described in Example 4, lactose (1200 mg), α-CD (150 mg) and purified water (12.5 mL) were added thereto, and then mixed (solids concentration 7.5%). This solution was spray-dried using an S.D.-GB22 to obtain a solidified fine particle-containing composition. As a result, a fine particle-containing composition was obtained, which contained 5 parts by weight of glibenclamide, 5 parts by weight of SDS, 10 parts by weight of α-CD and 80 parts by weight of lactose, based on 100 parts by weight of the fine particle-containing composition.

### (Test Example 3)

A storage test was performed and the average particle size was measured as in Test Example 1. The results are shown in Table 3. Even in a system including another excipient such as lactose, the cyclic oligosaccharide suppressed changes in particle size due to aggregation and crystal growth over time.

**[Table 3]**

| | Mixture ratio | Av. particle size (nm) | | Change (%) |
|---|---|---|---|---|
| | | Initial | After storage | |
| Comparative Example 1 | 5/5/0/90 | 210.7 | 885.5 | 320% |
| Example 5 | 5/5/5/85 | 218.5 | 532.5 | 144% |
| Example 6 | 5/5/10/80 | 227.2 | 301.4 | 33% |

| | | | | |
|---|---|---|---|---|
| Mixture ratio: glibenclamide/SDS/ α -CD/lactose | | | | |

### (Example 7)

Glibenclamide (2000 mg) was added to an aqueous SDS (20 mg/mL) solution (40 mL), which was stirred, and immediately transferred to a Nanomizer and then subjected to Nanomizer treatment for 10 minutes, so as to obtain a fine particle dispersion (hereinafter referred to as "dispersion C"). Based on dispersion C (2 mL), α-CD (1860 mg) and purified water (28 mL) were added thereto, and then mixed (solids concentration 6.7%). This solution was spray-dried using an S.D.-GB22 with an inflow air temperature of 115°C to obtain a fine particle-containing composition (air flow rate 0.5 m³/min, atomizing air 1 kgf/cm², liquid supply rate 7 mL/min). As a result, a fine particle-containing composition was obtained, which contained 5 parts by weight of glibenclamide, 2 parts by weight of SDS and 93 parts by weight of α-CD, based on 100 parts by weight of the fine particle-containing composition.

### (Example 8)

Based on dispersion C (2 mL) described in Example 7, α-CD (860 mg) and purified water (13 mL) were added thereto, and then mixed (solids concentration 6.7%). This solution was spray-dried using an S.D.-GB22 with an inflow air temperature of 115°C to obtain a fine particle-containing composition (air flow rate 0.5 m³/min, atomizing air 1 kgf/cm², liquid supply rate 7 mL/min). As a result, a fine particle-containing composition was obtained, which contained 10 parts by weight of glibenclamide, 4 parts by weight of SDS and 86 parts by weight of α-CD, based on 100 parts by weight of the fine particle-containing composition.

### (Example 9)

Based on dispersion C (2 mL) described in Example 7, α-CD (360 mg) and purified water (5.5 mL) were added thereto, and then mixed (solids concentration 6.7%). This solution was spray-dried using an S.D.-GB22 with an inflow air temperature of 115°C to obtain a fine particle-containing composition (air flow rate 0.5 m³/min, atomizing air 1 kgf/cm², liquid supply rate 7 mL/min). As a result, a fine particle-containing composition was obtained, which contained 20 parts by weight of glibenclamide, 8 parts by weight of SDS and 72 parts by weight of α-CD, based on 100 parts by weight of the fine particle-containing composition.

### (Example 10)

Based on dispersion C (2 mL) described in Example 7, α-CD (110 mg) and purified water (1.75 mL) were added thereto, and then mixed (solids concentration 6.7%). This solution was spray-dried using an S.D.-GB22 with an inflow air temperature of 115°C to obtain a fine particle-containing composition (air flow rate 0.5 m³/min, atomizing air 1 kgf/cm², liquid supply rate 7 mL/min). As a result, a fine particle-containing composition was obtained, which contained 40 parts by weight of glibenclamide, 16 parts by weight of SDS and 44 parts by weight of α -CD, based on 100 parts by weight of the fine particle-containing composition.

### (Control Example 2)

The dispersion C (solids concentration 7.0%) described in Example 7 was spray-dried as is using an S.D.-GB22 with an inflow air temperature of 115°C to obtain a fine particle-containing composition (air flow rate 0.5 m³/min, atomizing air 1 kgf/cm², liquid supply rate 7 mL/min).

### (Test Example 4)

A storage test was performed and average particle size was measured as in Test Example 1. The results are shown in Table 4. Not shown in the table is the average particle size of the particles in dispersion C, which was 675.6 nm. In comparison with Control Example 2 using no CD, in the examples using CD changes in particle size due to spray-drying were suppressed even at a high concentration (40%) of the drug. In particular, it was confirmed that at a drug concentration of from 5 to 20% the cyclic oligosaccharide was effective in suppressing changes in particle size due to aggregation and crystal growth over time in a solidified fine particle-containing composition containing the drug. This test also showed that as in the case of fine particle-containing compositions manufactured using dispersion A or dispersion B produced by dissolving the hardly-soluble drug in a good solvent in the mixing step, it was also possible to obtain a stable fine particle-composition using dispersion C produced by mixing and atomizing the hardly-soluble compound without dissolving it in a good solvent, although in this case the average particle size of the fine particles was slightly larger.

**[Table 4]**

| | Mixture ratio | Average particle size (nm) | | Change (%) |
|---|---|---|---|---|
| | | Initial | After storage | |
| Example 7 | 5/2/93 | 554.5 | 620.3 | 12% |
| Example 8 | 10/4/86 | 562.0 | 692.8 | 23% |
| Example 9 | 20/8/72 | 564.2 | 633.5 | 12% |
| Example 10 | 40/16/44 | 576.2 | 1325.2 | 130% |
| Control Example 2 | 71/29/0 | 624.5 | 7142.6 | 1044% |

| | | | | |
|---|---|---|---|---|
| Mixture ratio: glibenclamide/SDS/alpha-CD | | | | |

### (Example 11)

The fine particle-containing composition (100 mg) obtained in Example 1, lactose (400 mg), hydroxypropyl cellulose (15 mg) (HPC-L, Nippon Soda) and an aqueous α-CD (10%) solution (55 µL) were mixed in a mortar. This mixture was dried for 2 hours at 60°C, and then granulated by being forced through a #16 mesh sieve to obtain granules containing α-CD and fine particles of glibenclamide.

### (Example 12)

The fine particle-containing composition (100 mg) obtained in Example 2, lactose (400 mg), HPC-L (11.5 mg) and an aqueous HPC-L (7%) solution (54 µL) were mixed in a mortar. This mixture was first dried for 2 hours at 60°C and then granulated by being forced through a #16 mesh sieve to obtain granules containing β-CD and fine particles of glibenclamide.

### (Example 13)

The fine particle-containing composition (100 mg) obtained in Comparative Example 1, lactose (310 mg), α-CD (90 mg), HPC-L (15 mg) and an aqueous α-CD (10%) solution (55 µL) were mixed in a mortar. This mixture was first dried for 2 hours at 60°C and then granulated by being forced through a #16 mesh sieve to obtain granules containing α-CD and fine particles of glibenclamide.

### (Comparative Example 8)

The fine particle-containing composition (100 mg) obtained in Comparative Example 1, lactose (400 mg), HPC-L (15 mg) and purified water (50 µL) were mixed in a mortar. This mixture was first dried for 2 hours at 60°C and then granulated by being forced through a #16 mesh sieve to obtain granules containing lactose and fine particles of glibenclamide, but no CD derivatives.

### (Comparative Example 9)

The fine particle-containing composition (100 mg) obtained in Comparative Example 1, lactose (400 mg), HPC-L (11.5 mg) and an aqueous HPC-L (7%) solution (54 µL) were mixed in a mortar. This mixture was first dried for 2 hours at 60°C and then granulated by being forced through a #16 mesh sieve to obtain granules containing lactose and fine particles of glibenclamide, but no CD derivatives.

### (Test Example 5)

A storage test was performed as in Test Example 1. Average particle size was also measured as in Test Example 1. The results are shown in Table 5. As a result, it was shown that in the granules prepared using solidified fine particle-containing compositions, changes in particle size due to crystal growth and aggregation of fine particles of the hardly-soluble drug were suppressed even after the granulation operation. Surprisingly, an increase in particle size due to crystal growth and aggregation over time was almost completely prevented in Examples 11 to 13, which included cyclic oligosaccharide in the formulation. Example 13 is particularly remarkable since it was confirmed that the cyclic oligosaccharide is effective in preventing changes in particle size due to crystal growth and aggregation of nano-particles over time even when it was added at the granulation stage. That is, it was confirmed that an increase in the size of the fine particles of the hardly-soluble drug during storage can be prevented not only by adding the cyclic oligosaccharide to the fine particle dispersion before drying, but also by adding cyclic oligosaccharide to the solidified mixture (the first mixture in the third embodiment for example) after drying. This result demonstrates that the effect of the cyclic oligosaccharide in the present invention has a mechanism different from clathration of the hardly-soluble drug.

**[Table 5]**

| Component | | Example 11 | Example 12 | Example 13 | Comparative Example 8 | Comparative Example 9 |
|---|---|---|---|---|---|---|
| Glibenclamide | | 5 | 5 | 5 | 5 | 5 |
| SDS | | 5 | 5 | 5 | 5 | 5 |
| HPC-L | | 15 | 15.3 | 15 | 15 | 15.3 |
| α-CD or β-CD | | 95.5 | 90 | 95.5 | 0 | 0 |
| Lactose | | 400 | 400 | 400 | 490 | 490 |
| Total granules (mg) | | 521 | 515 | 521 | 515 | 515 |
| Av. particle size (nm) | Initial | 316.2 | 323.6 | 342.2 | 354.2 | 332.0 |
| | After storage | 311.9 | 308.8 | 338.8 | 464.1 | 397.6 |
| Change (%) | | -1% | -5% | -1% | 31% | 20% |

### (Control Example 3)

A DMSO solution (2 mL) of glibenclamide (200 mg/mL) was added to purified water (38 mL), which was stirred, and immediately transferred to a Nanomizer and then subjected to Nanomizer treatment. However, under preparation conditions without SDS the Nanomizer channel became blocked and a suspension could not be prepared. In Control Example 2, which included the surfactant, a fine particle dispersion containing fine particles having 1000 nm or less in size was obtained as well as a fine particle-containing composition after drying, confirming that in the manufacturing method of the present invention, the surfactant is particularly important during wet pulverization.

### (Test Example 6)

To confirm the effect of cyclodextrin, dispersions containing fine particles of glibenclamide were prepared by the following methods with varying proportions of SDS and cyclodextrin, and average particle size was compared. The average particle size was measured as in Test Example 1.

### (Preparation Methods)

A DMSO (2 mL) solution of glibenclamide (200 mg/mL) was added to an aqueous solution (38 mL) containing SDS (200 mg) and α-CD (169 mg), which was stirred, and immediately transferred to a Nanomizer and then subjected to Nanomizer treatment for 10 minutes, so as to obtain a fine particle-containing dispersion. Similarly, a DMSO solution (2 mL) of glibenclamide (200 mg/mL) was added to each (38 mL) of aqueous solutions containing 337 mg, 675 mg, 1350 mg and 2700 mg of α-CD, so as to obtain fine particle-containing dispersions.

### (Test Results)

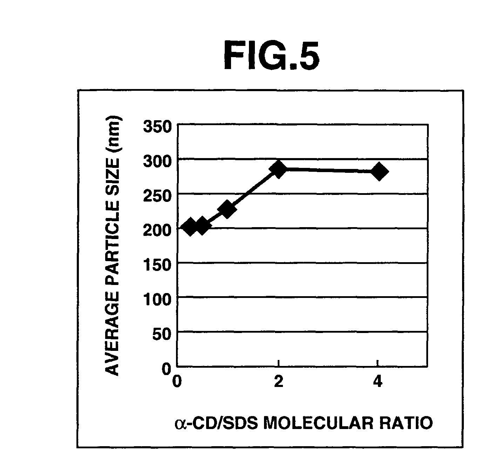

The test results are shown in Figure 5. It was shown that raising the concentration of α-CD relative to SDS during preparation of the fine particle-containing dispersion had a dramatic effect on the average particle size at a mole ratio of SDS to α-CD in the range of 1:1 to 1:2. This shows a diminished surfactant effect of SDS, suggesting the formation of a clathrate between the SDS and α -CD. This confirms that in order to obtain particles of a smaller size, it is desirable to first atomize a dispersion containing the drug and the surfactant, and then add cyclodextrin to clathrate the excess surfactant. Of course, the object of this application can also be achieved by adding the cyclodextrin in advance to a dispersion containing the drug and the surfactant.

### (Test Example 7)

Clathration of the surfactant using cyclodextrin and SDS was suggested in Test Example 6. This clathration was therefore investigated by means of a solubility measurement test in the presence of both.

### (Test Methods)

To an aqueous solution (4 mL) containing α-CD and SDS was added glibenclamide (20 mg), which was stirred for 3 days at room temperature. This solution was filtered using a Millex-HV filter (Millipore) with a pore size of 0.45 µm, and the glibenclamide concentration in the filtrate was measured by HPLC using a Unison UK-C18 column (Imtakt).

### (Test Results)

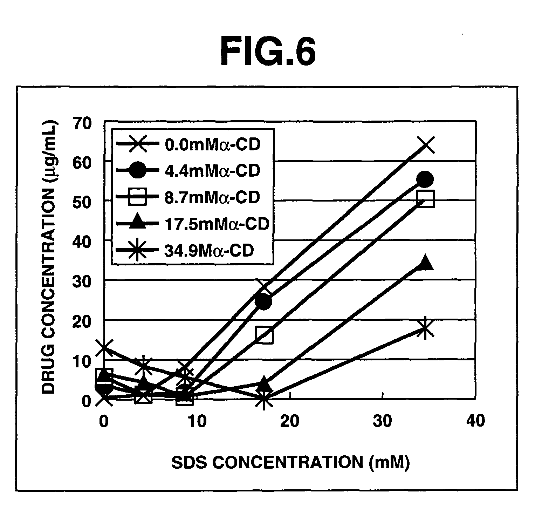

Figure 6 shows the solubility of glibenclamide relative to SDS concentration at various concentrations of α-CD. When the α -CD concentration was 0.0 mM, solubility rose as the SDS concentration rose, but when α-CD was added a bending point appeared indicating minimum solubility relative to SDS concentration. Such a solubility curve is an indicator of clathrate formation between the two. Moreover, this bending point corresponds to an α -CD/SDS mole ratio of 1 through 2 matching the results of Figure 5.

### (Example 14)

Based on dispersion A (7.5 mL), which was prepared by the methods described in Example 1, α-CD (1350 mg) and purified water (12.5 mL) were added thereto, and then mixed. The average particle size of the fine particles in the fine particle-containing dispersion of the drug in this case was 222.8 nm. This solution was spray-dried using an S.D.-GB22 with an inflow air temperature of 175°C to obtain a fine particle-containing composition (air flow rate 0.5 m³/min, atomizing air 1 kgf/cm², liquid supply rate 7 mL/min). As a result, a fine particle-containing composition was obtained, which contained 5 parts by weight of glibenclamide, 5 parts by weight of SDS and 90 parts by weight of α-CD, based on 100 parts by weight of the fine particle-containing composition. The average particle size of the fine particles in this fine particle-containing composition was 314.7 nm (175°C). This spray-drying temperature produced a slightly larger particle size than the temperature of 115°C used in Example 1 (with all other conditions the same), but a fine particle-containing composition containing fine particles having 1000 nm or less in size was still obtained.

### (Example 15)

Based on dispersion A (7.5 mL), which was prepared by the methods described in Example 1, β-CD (1350 mg) and purified water (92.5 mL) were added thereto, and then mixed (solids concentration 1.5%). The average particle size of the fine particles in the fine particle-containing dispersion of the drug in this case was 222.8 nm. This solution was spray-dried using an S.D.-GB22 with an inflow air temperature of 115°C (air flow rate 0.5 m³/min, atomizing air 1 kgf/cm², liquid supply rate 7 mL/min) to obtain a fine particle-containing composition. As a result, a fine particle-containing composition was obtained, which contained 5 parts by weight of glibenclamide, 5 parts by weight of SDS and 90 parts by weight of β -CD, based on 100 parts by weight of the fine particle-containing composition. The average particle size of the fine particles in this fine particle-containing composition was 407.9 nm. The particle size was somewhat large than in Example 2, in which the solids concentration of the fine particle-containing dispersion was 7.5%, but a fine particle-containing composition containing fine particles having 1000 nm or less in size was still obtained.

### (Example 16)

To an aqueous SDS (2 mg/mL) solution (40 mL) was added mefenamic acid (2-(2,3-dimethylphenylamino)benzoic acid (100 mg), which is practically insoluble in water, Wako Pure Chemical), which was stirred, and immediately transferred to a Nanomizer and then subjected to Nanomizer treatment for 10 minutes, so as to obtain a fine particle dispersion (hereinafter referred to as "dispersion X"). The average particle size of the drug particles in dispersion Z was 609 nm in this case. Based on dispersion X (1 mL) , α-CD (40 mg) was added thereto, and then mixed (solids concentration 4.4%). This solution was spray-dried using an S.D.-GB22 with an inflow air temperature of 115°C to obtain a fine particle-containing composition (air flow rate 0.5 m³/min, atomizing air 1 kgf/cm², liquid supply rate 7 mL/min). As a result, a fine particle-containing composition was obtained, which contained 5.6 parts by weight of mefenamic acid, 4.5 parts by weight of SDS and 89.9 parts by weight of α-CD, based on 100 parts by weight of the fine particle-containing composition.

### (Comparative Example 10)

Based on dispersion X (1 mL), which was prepared in Example 16, D-mannitol (40 mg) was added thereto, and then mixed (solids concentration 4.4%). This solution was spray-dried using an S.D.-GB22 with an inflow air temperature of 115°C to obtain a fine particle-containing composition (air flow rate 0.5 m³/min, atomizing air 1 kgf/cm², liquid supply rate 7 mL/min). As a result, a fine particle-containing composition was obtained, which contained 5.6 parts by weight of mefenamic acid, 4.5 parts by weight of SDS and 89.9 parts by weight of D-mannitol, based on 100 parts by weight of the fine particle-containing composition.

### (Example 17)

To an aqueous SDS (5 mg/mL) solution (40 mL) was added spironolactone (7α-acetylsulfanyl-3-oxo-17α -pregn-4-ene-21,17 β- carbolactone (100 mg), which is practically insoluble in water, Wako Pure Chemical), which was stirred, and immediately transferred to a Nanomizer and then subjected to Nanomizer treatment for 10 minutes, so as to obtain a fine particle dispersion (hereinafter referred to as "dispersion Y"). The average particle size of the fine drug particles in dispersion Z was 562 nm in this case. Based on this dispersion Y (1 mL), α-CD (42.5 mg) was added thereto, and then mixed (solids concentration 5%). This solution was then spray-dried using an S.D.-GB22 with an inflow air temperature of 115°C to obtain a fine particle-containing composition (air flow rate 0.5 m³/min, atomizing air 1 kgf/cm², liquid supply rate 7 mL/min). As a result, a fine particle-containing composition was obtained, which contained 5 parts by weight of spironolactone, 10 parts by weight of SDS and 85 parts by weight of α-CD, based on 100 parts by weight of the fine particle-containing composition.

### (Test Example 8)

A storage test was performed and average particle size was measured as in Test Example 1. However, the drug concentration for purposes of particle size measurement in this case was 0.1 mg/mL. The results are shown in Table 6. Table 6 shows that based on a comparison of Comparative Example 10 and Example 16 using SDS as the surfactant and mefenamic acid as the drug, an increase in particle size during storage under high-humidity conditions was strongly inhibited by mixing with α-CD. Although this is not shown in the table, almost no increase in particle size was also observed in Example 17 in which spironolactone was used as the drug and SDS as the surfactant, with an initial particle size after solidification being 756.1 nm and a particle size after storage being 826.9 nm (9% change). These results are surprising because they indicate that the particle size increase-inhibiting effect of α-CD can be obtained with various combinations of drug and surfactant.

**[Table 6]**

| | Sugar | Average particle size (nm) | | Change (%) |
|---|---|---|---|---|
| | | Initial | After storage | |
| Example 16 | α-CD | 664.3 | 735.3 | 11 % |
| Comparative Example 10 | D-mannitol | 615.8 | 1341 | 118% |

| | | | | |
|---|---|---|---|---|
| Mixture ratio: mefenamic acid/SDS/sugar = 5.6/4.5/89.9 | | | | |

To an aqueous SDS (4.2 mg/mL) solution (38 mL) was added a DMSO solution (2 mL) of glibenclamide (200 mg/mL), which was stirred, and immediately transferred to a Nanomizer and then subjected to Nanomizer treatment for 10 minutes, so as to obtain a fine particle dispersion. This dispersion was dialyzed with an aqueous SDS (4 mg/mL) solution to obtain a dispersion with DMSO removed (hereinafter referred to as "dispersion D"). The average particle size of the drug particles in dispersion Z in this case was 207 nm in this case. Based on dispersion D (1 mL), α-CD (186 mg) and purified water (1.67 mL) were added thereto, and then mixed (solids concentration 7.5%). This solution was spray-dried using an S.D.-GB22 with an inflow air temperature of 115°C to obtain a fine particle-containing composition (air flow rate 0.5 m³/min, atomizing air 1 kgf/cm², liquid supply rate 7 mL/min). A glibenclamide content of the fine particle-containing composition was 4.15 wt% as measured by high-performance liquid chromatography (detection wavelength 230 nm) using an ODS column with a 0.1 N potassium dihydrogenphosphate aqueous solution/acetonitrile = 9/11 mixture as the mobile phase. To this fine particle-containing composition (2318 mg) were added D-mannitol (5282 mg), corn starch (1000 mg), low-substituted hydroxypropyl cellulose (500 mg), hydroxypropyl cellulose (300 mg) and a suitable amount of purified water, which was mixed in a mortar, and then heat-dried in a thermostatic chamber. To the dried granules (8554 mg) were added low-substitution hydroxypropyl cellulose (455 mg) and magnesium stearate (91 mg). This mixture (130 mg) was tableted in an AG5000 A autograph (Shimadzu Corporation) to obtain a tablet with 7 mm in diameter, which contained 1.25 mg of glibenclamide.

### (Comparative Example 11)

To glibenclamide (96.2 mg) were added α-CD (2571.3 mg), D-mannitol (4894.0 mg), SDS (38.5 mg), corn starch (1000 mg), low-substitution hydroxypropyl cellulose (500 mg), hydroxypropyl cellulose (300 mg) and a suitable amount of purified water, which was mixed in a mortar, and then heat-dried using a thermostatic chamber. To the dried granules (8554 mg) were added low-substitution hydroxypropyl cellulose (455 mg) and magnesium stearate (91 mg). This mixture (130 mg) was tableted in an AG5000A autograph (Shimadzu Corporation) to obtain a tablet with 7 mm in diameter, which contained 1.25 mg of glibenclamide.]

### (Test Example 9)

A storage test was performed and average particle size was measured as in Test Example 1 using Example 18. In this test, the storage conditions were 60°C, 75% RH for 1 week and 1 month. Because the tablets contained insoluble additives, average particle size was measured after filtration using a filter with a pore size of 5 µm. As a result, the average particle size of the fine particles of glibenclamide in the tablets was 360.3 nm immediately after manufacture, 404.7 nm after 1 week of storage and 403.3 nm after 1 month of storage, for a change of 11 through 12% in average particle size after storage over the initial average particle size. The drug particles were stable even in tablets obtained by compression-molding using the fine particle-containing composition according to the present invention. This test confirms that the fine particles of the present invention are stable and are not affected by compression processes or excipients during manufacture or by storage conditions.

### (Test Example 10)

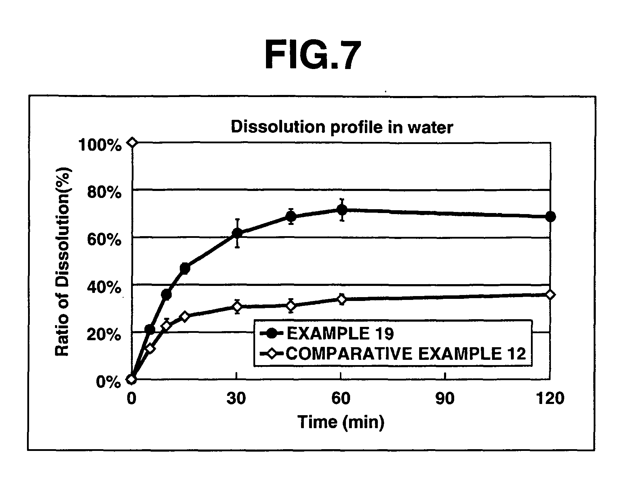

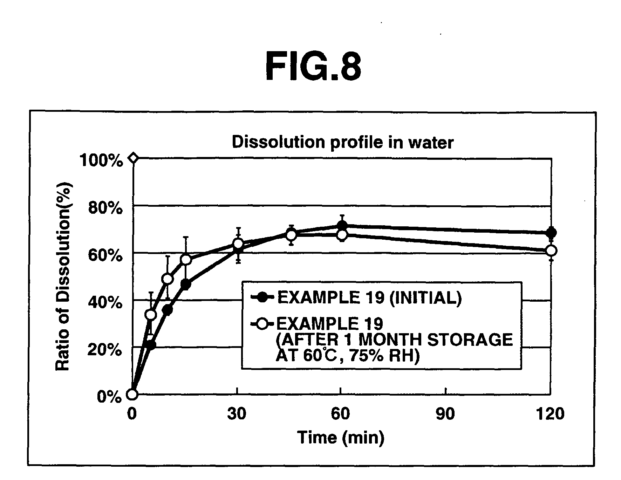

A dissolution test was performed with respect to Example 18 and Comparative Example 11. For Example 18, a dissolution test was also performed using the sample used in the storage test in Test Example 7. The dissolution test was performed in accordance with the dissolution test methods of the 14^{th} Japanese Pharmacopoeia, using water as the test liquid and a paddle rotation of 50 rpm. Sample liquid collected over time was first filtered with a Millipore PVDF filter (pore size 0.22 µm) and then subjected to HPLC analysis to calculate the glibenclamide concentration of the collected liquid and obtain the dissolution rate. HPLC analysis was performed using an ODS column with a detection wavelength of 230 nm using a 0.1 N potassium dihydrogenphosphate aqueous solution/acetonitrile = 9/11 mixture as the mobile phase. As a result, it was shown that drug dissolution was improved in Example 19, in which the drug was mixed as fine particles, as comparison to Comparative Example 12 in which the drug was mixed without being pulverized (see Figure 7). In Example 19, very little difference was seen between the dissolution profiles before and after storage (see Figure 8). This shows that the fine particle-containing composition according to the present invention provides improved dissolubility of the hardly-soluble drug, and that drug dissolution from the preparation does not change due to storage.

### Industrial Applicability

According to the present invention, the aggregation and crystal growth of fine particles of the hardly-soluble drug over time can be inhibited during the step of drying a suspension containing fine particles of the hardly-soluble drug and even in the dried composition through the additional use of cyclic oligosaccharide in the technique of pulverizing the hardly-soluble drug by wet pulverization using the surfactant. The present invention also provides the fine particle-containing composition in which fine particles of the hardly-soluble drug are physically stable and are not affected by environmental conditions such as temperature and humidity during storage. Consequently, according to the present invention, the fine particles of the hardly-soluble drug can be easily mixed into tablets and capsules, which are widely used as oral preparations, as well as into dry syrups and the like which can be re-dispersed in water and prepared as needed. Moreover, according to the present invention, the hardly-soluble drug can be orally administered with the size of the fine particles maintained, promoting absorption of the hardly-soluble drug, enhancing bioavailability, or allowing the dosage of the drug to be reduced, thereby providing the pharmaceutical composition with excellent compliance. In addition, because the fine particle-containing composition according to the present invention or the pharmaceutical composition containing the fine particle has excellent storage stability, it can be made widely available because it is easy to transport and distribute as a pharmaceutical premix material or as a pharmaceutical product.

## Claims

1. A fine particle-containing composition comprising a fine particle of a hardly-soluble drug, a surfactant and a cyclic oligosaccharide, wherein the fine particle has an average particle size in a range of from 50 nm to 1000 nm.

2. The fine particle-containing composition according to Claim 1, which is manufactured by a manufacturing method comprising the steps of:
(I) mixing the hardly-soluble drug, the surfactant and the poor solvent to obtain a liquid mixture;
(II) pulverizing the liquid mixture in a wet disperser to obtain a fine particle dispersion;
(III) adding the cyclic oligosaccharide to the fine particle dispersion; and
(IV) drying the fine particle dispersion containing the cyclic oligosaccharide.

3. The fine particle-containing composition according to Claim 1, which is manufactured by a manufacturing method comprising the steps of:
(I) mixing the hardly-soluble drug, the surfactant, the poor solvent and the cyclic oligosaccharide to obtain a liquid mixture;
(II) pulverizing the liquid mixture in a wet disperser to obtain a fine particle dispersion; and
(III) drying the fine particle dispersion.

4. The fine particle-containing composition according to Claim 1, which is manufactured by a manufacturing method comprising the steps of:
(I) mixing the hardly-soluble drug, the surfactant and a first poor solvent to obtain a liquid mixture;
(II) pulverizing the liquid mixture in a wet disperser to obtain a fine particle dispersion;
(III) first drying the fine particle dispersion to obtain a first mixture;
(IV) adding a cyclic oligosaccharide and a second poor solvent to the first mixture to obtain a second mixture; and
(V) second drying the second mixture.

5. The fine particle-containing composition according to any one of Claims 2 to 4, wherein the hardly-soluble drug is mixed in the mixing step as a solution of the hardly-soluble drug dissolved in a good solvent.

6. The fine particle-containing composition according to any one of Claims 2 to 5, wherein the manufacturing method comprises concentrating the fine particle dispersion or the fine particle dispersion containing the cyclic oligosaccharide prior to the drying step or the first drying step.

7. The fine particle-containing composition according to any one of Claims 2 to 6, wherein the drying step or the first drying step is a drying step employing a spray-drying.

8. The fine particle-containing composition according to any one of Claims 2 to 7, wherein the wet disperser is a homogenizer.

9. The fine particle-containing composition according to any one of Claims 1 to 8, wherein the surfactant is clathrated by the cyclic oligosaccharide.

10. The fine particle-containing composition according to any one of Claims 1 to 9, wherein the cyclic oligosaccharide comprises a cyclodextrin.

11. The fine particle-containing composition according to any one of Claims 1 to 10, wherein the surfactant comprises a surfactant having a hydrocarbon chain with 4 or more carbon atoms.

12. The fine particle-containing composition according to any one of Claims 1 to 11, wherein an amount of the hardly-soluble drug is from 0.1 to 40 parts by weight based on 100 parts by weight of the fine particle-containing composition.

13. A solid pharmaceutical composition comprising the fine particle-containing composition according to any one of Claims 1 to 13.

14. The solid pharmaceutical composition according to Claim 13, wherein the solid pharmaceutical composition is selected from the group consisting of a tablet, a granule, a capsules and a dry syrup.

15. A method for manufacturing a fine particle-containing composition, the method comprising the steps of:
(I) mixing a hardly-soluble drug, a surfactant and a poor solvent to obtain a liquid mixture;
(II) pulverizing the liquid mixture in a wet disperser to obtain a fine particle dispersion;
(III) adding a cyclic oligosaccharide to the fine particle dispersion; and
(IV) drying the fine particle dispersion containing the cyclic oligosaccharide,

16. A method for manufacturing a fine particle-containing composition, the method comprising the steps of:
(I) mixing a hardly-soluble drug, a surfactant, a poor solvent and a cyclic oligosaccharide to obtain a liquid mixture;
(II) pulverizing the liquid mixture in a wet disperser to obtain a fine particle dispersion; and
(III) drying the fine particle dispersion.

17. A method for manufacturing a fine particle-containing composition, the method comprising the steps of:
(I) mixing a hardly-soluble drug, a surfactant and a first poor solvent to obtain a liquid mixture;
(II) pulverizing the liquid mixture in a wet disperser to obtain a fine particle dispersion;
(III) first drying the fine particle dispersion to obtain a first mixture;
(IV) adding a cyclic oligosaccharide and a second poor solvent to the first mixture to obtain a second mixture; and
(V) second drying the second mixture.

18. The method for manufacturing the fine particle-containing composition according to any one of Claims 15 to 17, wherein the hardly-soluble drug is mixed in the mixing step as a solution of the hardly-soluble drug dissolved in a good solvent.

19. The method for manufacturing the fine particle-containing composition according to any one of Claims 15 to 18, further comprising concentrating the fine particle dispersion or the fine particle dispersion containing the cyclic oligosaccharide prior to the drying step or the first drying step.

20. The method for manufacturing the fine particle-containing composition according to any one of Claims 15 to 19, wherein the drying step or the first drying step is a drying step employing a spray-drying.

21. The method for manufacturing the fine particle-containing composition according to any one of Claims 15 to 20, wherein the wet disperser is a homogenizer.

22. The method for manufacturing the fine particle-containing composition according to any one of Claims 15 to 21, wherein the surfactant is clathrated by the cyclic oligosaccharide in the addition step.

23. The method for manufacturing the fine particle-containing composition according to any one of Claims 15 to 22, wherein the cyclic oligosaccharide comprises a cyclodextrin.

24. The method for manufacturing the fine particle-containing composition according to any one of Claims 15 to 23, wherein the surfactant comprises a surfactant having a hydrocarbon chain with 4 or more carbon atoms.

25. The method for manufacturing the fine particle-containing composition according to any one of Claims 15 to 24, wherein an amount of the hardly-soluble drug is from 0.1 to 40 parts by weight based on 100 parts by weight of the fine particle-containing composition.
